# EUROPEAN PATENT SPECIFICATION

(11) **EP 4 170 324 B1**
(45) Date of publication and mention of the grant of the patent: **13.08.2025**
(21) Application number: 21204232.9
(22) Date of filing: 22.10.2021
(51) Int. Cl.: G01N 21/78, G01N 21/84

(54) **METHOD AND SYSTEM FOR IMPROVED OPTICAL ANALYTE MEASUREMENTS**
VERFAHREN UND SYSTEM FÜR VERBESSERTE OPTISCHE ANALYTMESSUNGEN
PROCÉDÉ ET SYSTÈME DE MESURES D'ANALYTE OPTIQUE AMÉLIORÉES

(43) Date of publication of application: 26.04.2023
(73) Proprietor: F. Hoffmann-La Roche AG, 4070 Basel (CH); Roche Diabetes Care GmbH, 68305 Mannheim (DE)
(72) Inventor: BERG, Max, 68305 Mannheim (DE); HAILER, Fredrik, 68305 Mannheim (DE); LIMBURG, Bernhard, 68305 Mannheim (DE); STECK, Alexander, 68305 Mannheim (DE)
(74) Representative: Kierig, Elisabeth

(56) References cited:
- WO-A2-2014/025415
- US-A1- 2019 086 296
- US-A1- 2019 346 429

## Description

### TECHNICAL FIELD

The disclosure relates generally to the field of analyte measurement systems and, more specifically, to optical measurement systems that assist a user in performing an analyte measurement process.

### BACKGROUND

Analyte measurement systems that are known to the art enable the analysis of a bodily fluid dose provided by a user to identify the level of one or more analytes in the body of the user using an electronic device and one or more electrochemical reactions. These analyte measurement systems provide significant benefits for the accurate measurement of analytes in fluidic samples (i.e., biological or environmental) for individual users. Some analyte measurement systems employ a test strip that bears a chemical reagent. Upon receiving a fluid dose containing the analyte, a chemical reaction between the reagent and the analyte changes the color of the reagent, where the color change varies based on the concentration of the analyte, which in turn provides a measurement of the analyte. While many analytes are measured in this manner, one specific example of an analyte that is measured in a fluid dose is glucose, which is measured in a bodily fluid dose as part of monitoring and treatment of diabetes mellitus.

Older test strip systems that change color have relied upon a human observer to judge the analyte measurement by observing the change in color in the reagent, often with the assistance of printed color-matching guide. Such manual systems may present problems with reduced accuracy and inconsistent measurements based on the perceptions of different human observers. More recently, automated analyte measurement devices that use cameras to observe the reagent have been developed to provide improvements to analyte measurement accuracy. For example, widely available smartphones include optical sensors and digital image processing hardware that enables the smartphones to generate measurements of analytes in test strips when the smartphone executes a specifically configured analyte measurement software application.

While the use of specifically configured optical measurement devices improves the analyte measurement, challenges remain in ensuring the accuracy of the measurement process. One such challenge occurs in ensuring that optical measurements of a test strip are taken at the appropriate time after the test strip receives a dose of the fluid dose. An optical measurement that is taken too early may be inaccurate because the reagent has not had sufficient time to complete chemical reactions with the analyte, but if the optical measurement is taken too late then the reagent may have experienced drying or bleaching that affect the color of the reagent. Either situation may lead to inaccurate analyte measurement results even if the test strip and analyte measurement device are fully operable. Consequently, improvements to optical analyte measurement systems that overcome these challenges would be beneficial.

US 2019/0086296 A1 discloses a system for guiding collection of a hazardous contaminant sample comprising a substrate having a test area for the collection of the hazardous contaminant sample and a developing region, wherein one or more processors are configured to determine the time the liquid is applied to the acquisition pad using images captured by an imaging device and the developing region is scanned after a predetermined time after said application time.

US 2019/0346429 A1 discloses a system and method for automated camera-based optical assessment involving color assessment of a physical object, involving use of a specially-configured test card comprising a reagent pad configured to change to an expected color in response to an enzymatic reaction, a colored field with an the expected color and positioned adjacent to the reagent pad, and a QR code as an alignment as well as for verification and/or authentication.

### SUMMARY

The current invention concerns a method for measuring an analyte according to claim 1. The method includes identifying, with a processor, a test strip in a video stream generated by a camera based on at least one registration mark associated with the test strip depicted in the video stream, identifying, with the processor, application of a fluid dose to a deposit site formed on the test strip based on the video stream, activating, with the processor, a timer in response to the identification of the application of the fluid dose, generating, with an optical sensor, at least one optical measurement of a reagent located at a measurement site on the test strip, and generating, with the processor, a measurement of an analyte in the fluid dose based on the at least one optical measurement of the reagent only in response to the at least one optical measurement being generated after a predetermined minimum time period has elapsed subsequent to the activating of the timer and prior to a predetermined maximum time period elapsing subsequent to the activating of the timer.

According to the current invention, the method includes identifying, with the processor, a vial in a video stream generated by a camera based on at least one of an outline shape of the test vial or at least one registration mark located on the vial depicted in the video stream, identifying, with the processor, an opening of the vial in the video stream based on at least one registration mark located on a lid of the vial, and identifying, with a processor, extraction of the test strip from the vial after the identifying of the opening of the vial in the video stream based on the at least one registration mark associated with the test strip depicted in the video stream.

In a further embodiment of the method, the at least one registration mark associated with the vial further includes an indicator formed on a label of the vial.

In a further embodiment of the method, the at least one registration mark located on the lid of the vial further includes a color marking formed on an interior surface of the lid.

In a further embodiment of the method, the at least one registration mark associated with the test strip further includes a printed mark formed on a side of the test strip at a predetermined position relative to the deposit site.

In a further embodiment, the method includes identifying, with the processor, that a reverse side of the test strip is exposed in the video stream based on an absence of the printed mark formed on the side of the test strip and generating, with the processor and an output device, an output message indicating that the test strip should be rotated to expose the side of the test strip bearing the printed mark.

In a further embodiment of the method, the at least one registration mark associated with the test strip further includes an indicator formed on a rear surface of a color card that holds the test strip.

In a further embodiment, the method of identifying the application of the dose includes identifying, with the processor, a finger of a user in the video stream, and identifying, with the processor, contact between the finger and the deposit site in the video stream, and identifying, with the processor, the application of the dose in response to a change in an optical property of the deposit site in the video stream after the contact between the finger and the deposit site. In a further embodiment, the method of identifying the application of the dose further includes identifying, with the processor, the application of the dose in response to a change in an optical property of the deposit site in the video stream.

In a further embodiment, the method of identifying the application of the dose further includes identifying, with the processor, the application of the dose in response to contact between the finger and the deposit site in the video stream.

In a further embodiment, the method includes generating, with the processor and an output device, an output message informing the user that the measurement of the analyte in the fluid dose cannot be completed in response to no optical measurement of the measurement site being generated after the predetermined minimum time period has elapsed and prior to the predetermined maximum time period elapsing.

In a further embodiment of the method, the optical sensor that generates the measurement is the camera that generates the video stream.

In a further embodiment of the method, the optical sensor that generates the measurement is a camera that is different than the camera that generates the video stream.

In a further embodiment of the method, the camera is incorporated in a wearable electronic device and the optical sensor is incorporated in a mobile electronic device.

The current invention further concerns system for measurement of an analyte according to claim 14. The system includes a wearable electronic device and a mobile electronic device. The wearable electronic device includes a camera configured to generate the video stream and a transmitter configured to transmit the video stream to the mobile electronic device. The mobile electronic device includes a receiver configured to receive the video stream transmitted from the wearable electronic device, an optical sensor configured to generate optical measurements, a memory configured to store program instructions, and a processor operatively connected to the receiver, the optical sensor, and the memory. The processor is configured to execute the program instructions to identify a test strip in the video stream based on at least one registration mark associated with the test strip depicted in the video stream, identify application of a fluid dose to a deposit site formed on the test strip based on the video stream, activate a timer in response to the application of the fluid dose, generate, with the optical sensor, at least one optical measurement of a reagent located at a measurement site on the test strip, and generate a measurement of an analyte in the fluid dose based on the at least one optical measurement of the reagent only in response to the at least one optical measurement being generated after a predetermined minimum time period has elapsed subsequent to the activating of the timer and prior to a predetermined maximum time period elapsing subsequent to the activating of the timer.

According to the current invention, the processor is further configured to identify a vial in the video stream generated by a camera based on at least one registration mark located on the vial depicted in the video stream, identify an opening of the vial in the video stream based on at least one of an outline shape of the test vial or at least one registration mark located on a lid of the vial, and identify extraction of the test strip from the vial after the identification of the opening of the vial in the video stream based on the at least one registration mark associated with the test strip depicted in the video stream.

In a further embodiment of the system, the at least one registration mark associated with the vial further includes an indicator formed on a label of the vial.

In a further embodiment of the system, the at least one registration mark located on the lid of the vial further includes a color marking formed on an interior surface of the lid.

In a further embodiment of the system, the at least one registration mark associated with the test strip further includes an indicator formed on a surface of the test strip at a predetermined position relative to the deposit site.

In a further embodiment, the processor is configured to identify that a reverse side of the test strip is exposed in the video stream based on an absence of the indicator formed on the surface of the test strip, and generate, with an output device, an output message indicating that the test strip should be rotated to expose the surface of the test strip bearing the indicator.

In a further embodiment of the system, the at least one registration mark associated with the test strip further includes an indicator formed on a rear surface of a color card that holds the test strip.

In a further embodiment, the processor is configured to identify a finger of a user in the video stream, identify contact between the finger and the deposit site in the video stream, and identify the application of the dose in response to a change in an optical property of the deposit site in the video stream after the contact between the finger and the deposit site

In a further embodiment, the processor is configured to identify the application of the dose in response to a change in an optical property of the deposit site in the video stream.

In a further embodiment, the processor is configured to identify the application of the dose in response to contact between the finger and the deposit site in the video stream.

In a further embodiment, the system includes an output device in at least one of the wearable electronic device or the mobile electronic device, the processor being operatively connected to the output device and further configured to generate an output message informing the user that the measurement of the analyte in the fluid dose cannot be completed in response to no optical measurement of the measurement site being generated after the predetermined minimum time period has elapsed and prior to the predetermined maximum time period elapsing.

### BRIEF DESCRIPTION OF THE DRAWINGS

The advantages, effects, features and objects other than those set forth above will become more readily apparent when consideration is given to the detailed description below. Such detailed description makes reference to the following drawings, wherein:
- FIG. 1: is a diagram depicting components in an analyte measurement system that uses a wearable electronic device and a mobile electronic device to identify when a test strip receives a dose during an analyte measurement process.
- FIG. 2: is a schematic diagram depicting components of the wearable electronic device and mobile electronic device of FIG. 1.
- FIG. 3: is a block diagram of a process for the operation of the analyte measurement system.
- FIG. 4: is a series of views of a vial that holds test strips as the vial is opened and a test strip is removed from the vial.
- FIG. 5: is a series of views of a test strip that receives a fluid dose.
- FIG. 6: is a series of views of a test strip that is placed in a color card to receive a fluid dose.

### DETAILED DESCRIPTION

These and other advantages, effects, features and objects are better understood from the following description. In the description, reference is made to the accompanying drawings, which form a part hereof and in which there is shown by way of illustration, not limitation, embodiments of the inventive concept. Corresponding reference numbers indicate corresponding parts throughout the several views of the drawings.

While the inventive concept is susceptible to various modifications and alternative forms, exemplary embodiments thereof are shown by way of example in the drawings and are herein described in detail. It should be understood, however, that the description of exemplary embodiments that follows is not intended to limit the inventive concept to the particular forms disclosed, but on the contrary, the intention is to cover all advantages, effects, and features falling within the scope of the invention as defined by the appended claims.

As such, it should be noted that the embodiments described herein may have advantages, effects, and features useful in solving other problems.

The devices, systems and methods now will be described more fully hereinafter with reference to the accompanying drawings, in which some, but not all embodiments of the inventive concept are shown. Indeed, the devices, systems and methods may be embodied in many different forms and should not be construed as limited to the embodiments set forth herein; rather, these embodiments are provided so that this disclosure will satisfy applicable legal requirements.

Likewise, many modifications and other embodiments of the devices, systems and methods described herein will come to mind to one of skill in the art to which the disclosure pertains having the benefit of the teachings presented in the foregoing descriptions and the associated drawings. Therefore, it is to be understood that the devices, systems and methods are not to be limited to the specific embodiments disclosed and that modifications and other embodiments are intended to be included within the scope of the embodiments. Although specific terms are employed herein, they are used in a generic and descriptive sense only and not for purposes of limitation.

Unless defined otherwise, all technical and scientific terms used herein have the same meaning as commonly understood by one of skill in the art to which the disclosure pertains. Although any methods and materials similar to or equivalent to those described herein can be used in the practice or testing of the methods, the preferred methods and materials are described herein. Moreover, reference to an element by the indefinite article "a" or "an" does not exclude the possibility that more than one element is present, unless the context clearly requires that there be one and only one element. The indefinite article "a" or "an" thus usually means "at least one." Likewise, the terms "have," "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. For example, the expressions "A has B," "A comprises B" and "A includes B" may refer both to a situation in which, besides B, no other element is present in A (*i.e.,* a situation in which A solely and exclusively consists of B) or to a situation in which, besides B, one or more further elements are present in A, such as element C, elements C and D, or even further elements.

As used herein, the term "mobile electronic device" refers to a portable computing device that provides a user one or more of each of the following components: an output device, an input device, a memory, and a wireless communication device that are controlled by one or more processors in the mobile electronic device. As used herein, the term "wearable electronic device" refers to a type of mobile electronic device that is further adapted to be worn by a human user in a similar manner to glasses, clothing, watches, or jewelry. Examples of output devices include, but are not limited to, liquid crystal display (LCD) displays, organic or inorganic light emitting diode (LED) displays, and other forms of graphical display device, audio speakers, and haptic feedback devices. Examples of input devices include, but are not limited to buttons, keyboards, touchscreens, and audio microphones. Examples of memory include, but are not limited to, both volatile data storage devices such as random-access memory (RAM) and non-volatile data storage devices such as magnetic disks, optical disks, and solid-state storage devices including EEPROMs, NAND flash, or other forms of solid-state data storage devices. Examples of wireless communication devices include, but are not limited to, radio transceivers that operate with the Near Field Communication (NFC) protocol, the Bluetooth protocol family, including Bluetooth Low Energy (BLE), the IEEE 802.11 protocol family ("Wi-Fi"), and cellular data transmission standards ("4G," "5G," or the like). Examples of the processors include digital logic devices that implement one or more central processing units (CPUs), graphics processing units (GPUs), neural network processors (NPUs), digital signal processors (DSPs), field programmable gate arrays (FPGAs), application specific integrated circuits (ASICs), and any other suitable digital logic devices in an integrated device or as a combination of devices that operate together to implement the processor. Common examples of mobile electronic devices include, but are not limited to, smartphones, tablet computing devices, and notebook computers. Common examples of wearable electronic devices include, but are not limited to, smart watches and smart glasses.

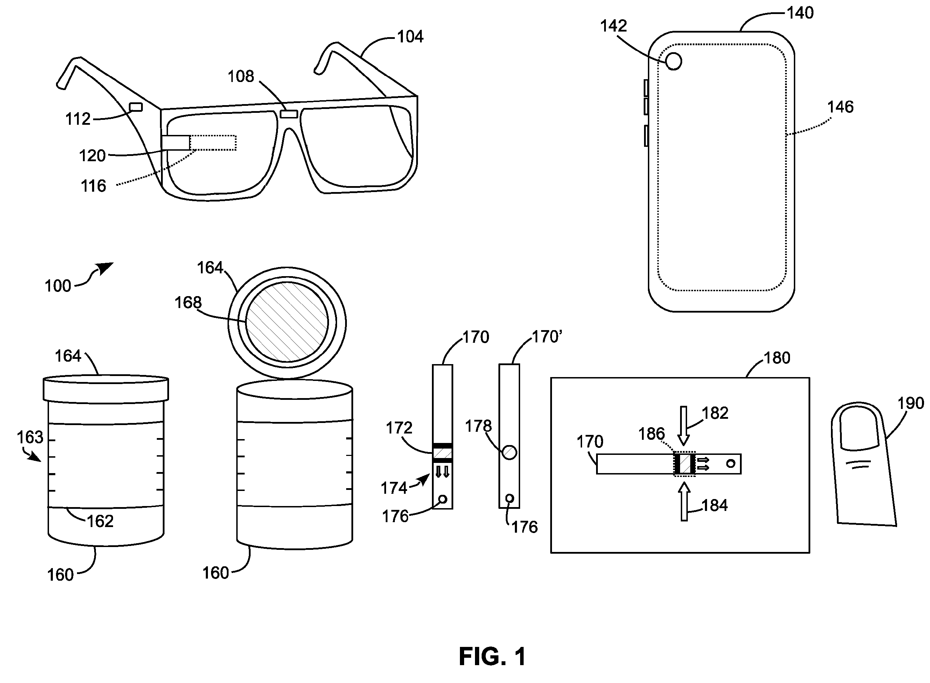

FIG. 1 depicts an analyte measurement system 100 that includes a wearable electronic device 104 and a mobile electronic device 140. During operation, the wearable electronic device 104 generates a video stream that enables the mobile electronic device 140 to track a vial 160 that holds one or more test strips 170 to identify when the test strip 170 receives a fluid dose, such as a blood dose from a finger 190 of human test subject. As described in further detail below, the system 100 starts a timer upon detection of application of a fluid dose to a deposit site 172 on one side of the test strip 170 to enable an optical sensor 142 in the mobile electronic device 140 to generate optical measurements of a measurement site 178 on a test strip reverse side 170' after a predetermined minimum time has elapsed and prior to a predetermined maximum time elapsing.

In FIG. 1, the test strip 170 includes a deposit site 172 where a user provides a liquid blood sample. The test strip 170 also includes registration marks 174, which are depicted as printed marks in the form of indicator arrows on the surface of the test strip 170 at a predetermined position relative to the deposit site 172. A hole 176 is also formed through one end of the test strip 170. The registration marks 174 enable efficient identification and tracking of the test strip 170, including identification of which side of the test strip 170 faces the camera in the video stream. In the illustrative example of FIG. 1, only one side of the test strip 170 is configured to receive the blood sample and the registration marks 174 are formed only on this side of the test strip 170, although in alternative embodiments a test strip may be configured to receive a blood dose on either side of the test strip. The test strip reverse side 170' refers to the same test strip 170, but with the reverse side of the test strip in view including the measurement site 178 and the hole 176. Neither the registration mark arrow indicators 174 nor the deposit site 172 are visible to the camera 108 when the test strip reverse side 170' is in view. In some embodiments, the system 100 can detect that the test strip reverse side 170' is exposed to the camera 108. In the test strip 170, the deposit site 172 provides a fluid inlet that enables a fluid dose to permeate through one or more internal layers in the test strip to enable chemical reactions with one or more reagents in the test strip. Examples of internal layers include, for example, filters and different layers of chemical reagents that react to one or more analytes in the fluid dose. The measurement site 178 is an optically exposed region formed on the test strip reverse side 170' that changes color in response to the level of analyte in the fluid dose that permeates through the test strip from the deposit site 172. In one configuration, the reagent is exposed at the measurement site 178 directly, while in another configuration an optically transparent layer, such as a film, covers the reagent while providing an optical aperture. Both configurations enable generation of optical measurements of the reagent to detect changes in the color of the reagent due to reaction with the analyte in the fluid dose.

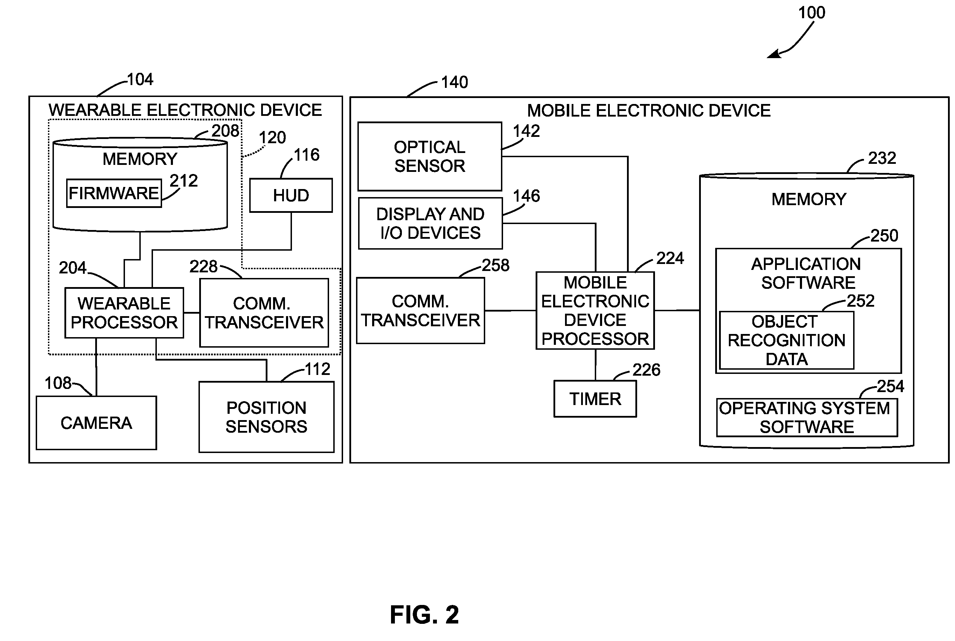

Referring to FIG. 1 and to a schematic diagram in FIG. 2, in one embodiment of the system 100 the wearable electronic device 104 is embodied as a pair of eyeglasses, also referred to as "smart glasses", although other forms of wearable electronic devices including smart watches may be used in alternative configurations. The wearable electronic device 104 includes a frame and optional lenses that are similar to a conventional pair of eyeglasses. The wearable electronic device 104 further includes a camera 108, positional sensors 112, and a head-up display (HUD) 116, that are each operatively connected to an electronic control unit 120. The camera 108 is, for example, a CMOS or other suitable digital imaging device that generates images and video streams of the region in front of the wearable electronic device 104 that corresponds to the view of the person wearing the wearable electronic device 104. In some embodiments, a single monochrome or color camera generates the video stream as a two-dimensional video stream. In other embodiments, the camera 108 is further configured to generate a video stream that provides three-dimensional object data. In one configuration, the camera 108 further incorporates two or more cameras that provide stereoscopic video, and, in another configuration, the camera 108 includes a depth sensor that provides three-dimensional depth information corresponding to objects in the video stream. The positional sensors 112 include, for example, a microelectromechanical (MEMs) three-axis gyroscope and one or more accelerometers that provide data to identify the spatial orientation of the wearable electronic device 104 during operation. The HUD 116 provides visual outputs to the wearer without requiring the wearer to change his or her gaze to a particular display device. While FIG. 1 depicts a HUD 116 that is separate from the glass lenses in the wearable electronic device 104, alternative configurations provide one or more visual display devices that are integrated into the lenses or that project graphical outputs onto the lenses. While not depicted in further detail, the wearable electronic device 104 optionally includes audio output devices as well.

The electronic control unit 120 houses at least one wearable electronic device processor 204 that is operatively connected to the camera 108, the positional sensors 112, and the HUD 116. The electronic control unit 120 further houses a memory 208 and a communications transceiver 228 that are operatively connected to the wearable electronic device processor 204. In the embodiment of FIG. 2, the memory 208 stores firmware instructions 212 that control the operation of the wearable electronic device 104. The communications transceiver 228 includes a transmitter that enables transmission of data, including a video stream, to a corresponding communications transceiver 258 in the mobile electronic device 140. The communications transceiver 228 further includes a receiver that enables the wearable electronic device 104 to receive data from the mobile electronic device 140, and, in particular, to receive messages from the mobile electronic device 140 for display to a user via the HUD 116. In the illustrative example of FIG. 2, the communications transceiver 228 is a Bluetooth or Bluetooth low-energy wireless data communications transceiver, although alternative configurations may use a different wireless communication standard or may employ a wired connection interface such as Universal Serial Bus (USB).

Referring to FIG. 1 and to a schematic diagram in FIG. 2, in one embodiment of the system 100 the mobile electronic device 140 further includes a mobile electronic device processor 224 that is operatively connected to a timer 226, a memory 232, a communication transceiver 258, the optical sensor 142, and one or more display and user input/output (I/O) devices 146. The mobile electronic device 140 is operatively connected to the wearable electronic device 104 using the transceiver 258 to communicate with the corresponding transceiver 228 in the wearable electronic device 104.

In the mobile electronic device 140, the optical sensor 142 is, for example, a digital camera that generates still images or video of the test strip 170, including the measurement site 178 located on the test strip reverse side 170', and, optionally, the color card 180 to generate at least one optical measurement for analysis to measure the analyte level in the fluid dose that is applied to the test strip. While the optical sensor 142 and the camera 108 in the wearable electronic device 104 may be configured with similar hardware in some embodiments, in the configuration of FIG. 1 the camera 108 is configured for the generation of a video stream that captures the overall scene including the vial 160, the test strip 170 and the deposit site 172, optionally the color card 180, and the finger 190 for identification of the time at which the test strip 170 receives a fluid dose. The optical sensor 142 is configured to generate one or more optical measurements of the measurement site 178 on the test strip reverse side 170' at the appropriate time after application of the fluid dose to provide an input to an analyte measurement process. The optical measurement is, for example, a digital photograph that includes the measurement site 178 on the test strip reverse side 170'. As such, the camera 108 provides a broader view of multiple elements employed in the analyte testing process, while the optical sensor 142 provides more detailed digital images or video of the reagent on the test strip 170 and optionally the calibration data that are provided on the color card 180. In an alternative configuration, a single camera performs both the functions of the camera 108 in the wearable electronic device 104 and the optical sensor 142 in the mobile electronic device 140. For example, a single camera may be reconfigurable to generate a video stream with a reduced resolution for identification and tracking of the vial 160, the test strip 170, the color card 180, and the finger 190 to enable the mobile electronic device 140 to identify the time at which the test strip 170 receives the fluid dose. Subsequently, the single camera may operate at a higher resolution to capture one or more high-fidelity images of the test strip reverse side 170' either alone or in conjunction with the color card 180 to provide an input for the analyte measurement process.

In the mobile electronic device 140, the user input/output (I/O) devices 146 include a touchscreen display device that provides a graphical output to a user and receives touch inputs to control the operation of the mobile electronic device 140 and, more particularly, to provide input to the analyte measurement process. Other examples of I/O devices include microphones for speech input and speakers for audio output, mechanical buttons, and the like. In some configurations, the wearable electronic device 104 implements user I/O devices such as an audio input device or gesture tracking input device that uses the camera 108 to record inputs from the user that the camera 108 transmits to the mobile electronic device 140. The wearable electronic device 104 may further receive output data from the mobile electronic device 140 for display to the user via the HUD 116.

In the mobile electronic device 140, the timer 226 enables the mobile electronic device processor 224 to maintain a count of elapsed time during operation, which includes counting an elapsed time starting at when the test strip 170 receives a fluid dose to ensure that optical measurements of the reagent for analyte measurement occur after a predetermined minimum time has elapsed and before a predetermined maximum time has elapsed. While the timer 226 is depicted as a discrete component for illustrative purposes, in many practical embodiments the timer 226 is integrated into the mobile electronic device processor 224 as a timer circuit or is implemented as a software timer.

In the mobile electronic device 140, the memory 232 includes one or more non-volatile and volatile data storage devices. In the configuration of FIG. 2, the memory 232 stores application software 250 and operating system software 254 that both contain instructions for execution by the mobile electronic device processor 144. The application software 250 includes instructions that implement a user interface and an analyte analysis program to perform the analyte measurement process based on an image analysis of one or more optical measurements of the reagent on the test strip 170. The application software 250 also stores predetermined minimum and maximum elapsed time thresholds to ensure that the optical measurements are generated after the fluid dose has had sufficient time to react with the reagent in the test strip 170 but before a maximum useful time period for measuring the analyte has elapsed. As described in further detail below, part of the analyte measurement process includes the identification of the vial 160, removal of the test strip 170, and the identification of contact between the finger 190 and the test strip 170 to apply the fluid dose to the deposit site 172. The application software 250 further includes object recognition data 252 that enable the mobile electronic device processor 224 to perform automated object identification and tracking of the vial 160 and test strip 170 in the video stream that is received from the wearable electronic device 104. The object recognition data 252 are generated through a training process that occurs prior to distribution of the application software 250. In particular, the training process utilizes the predetermined shapes, colors, and patterns of registration marks formed on the vial 160, test strip 170, test strip reverse side 170', and the color card 180 to enable automated identification and tracking of these components in a video stream. Examples of the object recognition data 252 include image classifiers such as neural networks, particularly convolutional neural networks, support vector machines, hidden Markov models, one-dimensional and two-dimensional barcode scanning engines, and the like. Additionally, the object recognition data 252 may include filters for color detection and edge detection along with other image processing data needed for tasks such as object detection and image segmentation to enable tracking of objects such at the vial 160, test strip 170, and color card 180 in the video stream. The operating system (OS) software 254 includes the software kernel, drivers, libraries, and other system software that are associated with a standard commercially-available operating system. The OS software 254 provides standardized services such as network and graphics stacks, file systems for data storage and management, software access to the optical sensor 142, display and I/O devices 146, timer 226, communications transceiver 258, and other components in the mobile electronic device 140. In the mobile electronic device 140, the communications transceiver 258 includes a transmitter that enables transmission of data, including command data and output message data, to the corresponding transceiver 228 in the wearable electronic device 104. The communications transceiver 258 further includes a receiver that enables the mobile electronic device 140 to receive data from the mobile electronic device 140, and, in particular, to receive a video stream from the camera 108 in the wearable electronic device 104. In the illustrative example of FIG. 2, the communications transceiver 258 is a Bluetooth or Bluetooth low-energy wireless data communications transceiver, although alternative configurations may use a different wireless communication standard or may employ a wired connection interface such as USB.

FIG. 1 further depicts a vial 160 and a color card 180. The vial 160 stores one or more of the test strips 170. In addition to providing storage, the vial 160 protects the test strips from contamination in the environment, which includes preventing the reagents in the test strips 170 from absorbing excessive amounts of moisture and light from the ambient environment. The vial 160 includes a printed label 162 that further bears one or more registration marks, which are depicted as the dashed line indicator registration marks 163 that are printed along one or more edges of the label 162 in FIG. 1, or another suitable surface of the vial 160 in another embodiment. The outline shape of the vial 160 and the registration marks 163 form a simple visual indicator that enable efficient identification and tracking of the vial 160 in a video stream that the wearable electronic device 104 generates and transmits to the mobile electronic device 140. The registration marks 163 are widely distributed on the exterior of the vial 160 to enable identification and tracking of the vial 160 from a wide range of viewing angles and when the hand of a user holds the vial 160. Alternative embodiments of registration marks for the vial 160 include, for example, printed pattern indicators including barcodes, or engraved or embossed geometric shapes that are formed on the exterior of the vial 160 that assist in automated identification and tracking of the vial 160. In the embodiment of FIG. 1, a lid 164 provides access to the interior of the vial 160. The lid 164 may be fully removable or may remain attached to the body of the vial 160 while opened. In either configuration, a second registration mark 168 is formed on the interior surface of the vial lid 164. The second registration mark 168 is, for example, a circle or other geometric shape formed with a predetermined color that contrasts with the color of the vial 160 to provide a clear indication that the vial 160 has been opened in the video stream that the wearable electronic device 104 generates during use of the vial 160. In alternative embodiments, the registration mark 168 is a one or two dimensional barcode or other registration mark that is identifiable to automated vision algorithms. The registration marks 163 and 168 enable accurate identification of both the vial 160 and a determination of when the vial 160 is closed or opened.

During operation, the optical sensor 142 detects the color change in the reagent that is visible to the measurement site 178 on the test strip reverse side 170' in response to one or more chemical reactions with the analyte in the fluid dose. In the illustrative example of FIG. 1, the change in color of the reagent located at the measurement site 178 indicates a level of glucose analyte in the blood sample. As described above, the system 100 identifies when the deposit site 172 receives the fluid dose and uses a timer to determine when an optical sensor should generate subsequent optical measurements of the measurement site 178 to ensure an accurate measurement of the blood glucose level. While the system 100 depicts a test strip that includes a separate deposit site 172 and measurement site 178 for illustrative purposes, those of skill in the art will recognize that alternative test strips provide a single deposit site and measurement site with the reagent that are co-located on the same region of the test strip. As such, in some embodiments the deposit site and reagent occupy separate locations on the test strip while in other embodiments the deposit site and the reagent refer to a single location of the test strip. In FIG. 1, the color card 180 is an optional component that has a rear side depicted in FIG. 1 that holds the test strip 170 in place prior to dosing. The color card 180 also has a front side (not shown) that includes a predetermined arrangement of colors and other fiducial markings that assist in calibrating images from the optical sensor 142 for accurate color measurement of the exposed measurement site 178. An aperture 186 in the color card 180 enables the measurement site 178 on the test strip reverse side 170' to be measured by the optical sensor within the color pattern of the color card 180. The rear side of the color card 180 includes registration marks 182 and 184, which are depicted as arrow indicators that are printed on the rear side of the color card 108 in the illustrative embodiment of FIG. 1. The registration marks 182 and 184 are associated with the test strip 170 and further assist in identifying and tracking the test strip 170 in the video stream to detect when the test strip 170 receives the fluid dose. The color card 180 is optional, and the system 100 is configured to generate optical measurements of the reagent at the measurement site 178 in the test strip reverse side 170' to measure the glucose analyte in a blood sample using the test strip reverse side 170' in isolation or in conjunction with the color card 180.

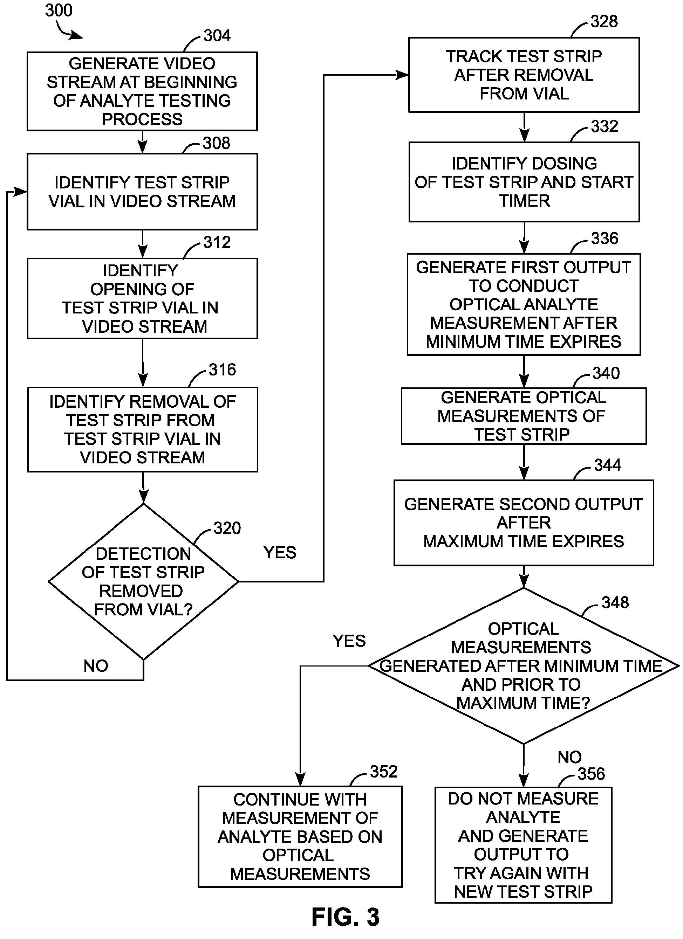

FIG. 3 depicts a process 300 for operation of the system 100 to perform an analyte testing operation with automated detection of when a fluid dose is applied to the deposit site of a test strip and automated timing of when the system 100 should generate one or more optical measurements of the measurement site to measure a level of analyte in the fluid dose. In the description of the process 300, a reference to the process performing a function or action refers to the operation of one or more digital processors, such as the processors in the wearable electronic device 104 and the mobile electronic device 140, to execute stored program instructions to perform the function or action in conjunction with other components in the system 100.

The process 300 begins with activation of a camera, such as the camera 108 in the wearable electronic device 104, to generate a video stream of a scene in front of a user at the beginning of an analyte testing process (block 304). In the system 100, the user begins execution of the application software 250, and the mobile electronic device 140 transmits a command to the wearable electronic device 104 to activate the camera 108. In the embodiment of FIG. 1, the wearable electronic device processor 204 activates the camera 108 and uses the transceiver 228 to transmit a video stream from the camera 108 to the mobile electronic device 140, which enables the mobile electronic device processor 224 to receive the video stream for further processing using the corresponding transceiver 258. As is generally known in the art, the video stream includes a series of frames of image data that depict the view from the camera 108 over time during the analyte testing process.

The process 300 continues as the mobile electronic device processor 224 identifies the vial 160 in the video stream generated by the wearable electronic device 104 (block 308). While numerous digital image processing techniques may be used to identify an object, such as the vial 160 or other objects that are detected in the video stream during the process 300, a nonlimiting example of the preferred technique is described in further detail herein. The identification process for the vial 160 further includes an object tracking operation that segments different portions of frames in the video stream that contain objects and an object identification operation that uses an image classifier to identify the tracked objects.

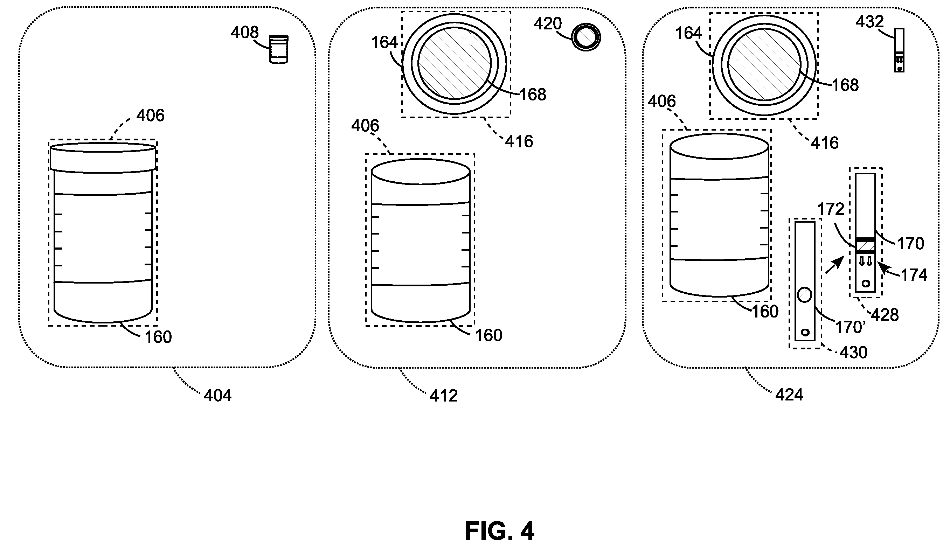

In the object tracking operation, the mobile electronic device processor 224 in the mobile electronic device 140 identifies and tracks one or more objects that are depicted in the video stream. To track objects, the mobile electronic device processor 224 performs a contour detection operation that identifies the boundaries of various objects in the video stream that have similar image intensity values, including the boundaries of the vial 160. In particular, each frame of the video stream is formed as a two-dimensional array of pixels, and the mobile electronic device processor 224 identifies contours based on contiguous regions of pixels with the same or similar numeric pixel values in either color data (e.g. red/green/blue) or monochrome image data (e.g. gray scale values). In some configurations, the mobile electronic device processor 224 performs image pre-processing operations such as converting a color video stream to grayscale, thresholding of the grayscale pixels, and performing an edge detection processing to improve the accuracy of the contour detection process. The mobile electronic device processor 224 segments the original image using, for example, rectangular bounding boxes that surround the detected contour areas, and the mobile electronic device processor 224 performs the contour detection process over a series of video frames to track the movement of the object, such as when a user moves the vial 160. For example, as depicted in view 404 of FIG. 4, the video stream depicts the vial 160 and the mobile electronic device processor 224 generates a rectangular bounding box 406 segment in a frame of the video stream that contains the detected contour of vial 160. While view 404 depicts the vial 160 in isolation, some frames in the video stream contain more than one object and the contour detection process described above enables tracking of multiple objects in the video stream.

Upon completion of the tracking operation, the mobile electronic device processor 224 has access to one or more image segments that contain objects, but has not yet determined the identity of specific objects. For example, the mobile electronic device processor 224 has tracked an object in the image segment 406 but has not yet identified that the object is the vial 160 or some other object. The object tracking process produces multiple image segments that can improve the accuracy of the image classifier for the detection of multiple relevant objects that may occupy different portions of a frame in the video stream. To complete the object identification process, the mobile electronic device processor 224 provides the segmented portion of the image containing the tracked object as an input to a trained image classifier that is stored with the object recognition data 252 in the memory 232. The image classifier is, for example, a trained convolutional neural network (CNN) or other suitable image classifier that is trained to identify a predetermined set of objects, such as the vial 160 and interior of the vial lid 164, either side of the test strip 170/170', the color card 180, or a finger 190. The training process for the image classifier occurs prior to the process 300 and uses a set of training images that include multiple examples of the objects to be identified in various expected situations that would occur during an analyte testing process. The image classifier is trained using, for example, a gradient descent training process that is otherwise known to the art. The image classifier is trained to recognize, either expressly or implicitly, some or all of the outline shape of the vial 160, registration mark features 163 that are formed on the vial 160, the interior of the vial lid 164, on either side of the test strip 170/170', and on the color card 180 to improve the accuracy of identifying the predetermined objects. Additionally, the training process can include training examples that occur when the registration marks are only partially visible to the camera 108, such as when a user holds the vial 160 in hand, which may occlude some of the registration marks 163. The mobile electronic device processor 224 optionally performs additional pre-processing of the image data, which may include resizing the image data to a predetermined resolution, or performing a rotational transformation of the image based on metadata that are received from the positional sensors 112 in the wearable electronic device 104 that identifies the angular orientation of the camera 108 at the time each frame of the video stream is generated to improve the accuracy of the image classifier. In some configurations, the image classifier is trained using monochrome image data, but in other configurations, a color image is preferred, including configurations in which registration marks are formed using predetermined colors that assist in image classification to identify an object. The classifier also rejects extraneous objects that may be present in the video stream as non-relevant. Additionally, because the video stream includes a series of frames, the mobile electronic device 140 can recognize the vial 160 in one or more frames of the video stream even if the tracking and identification process is not successful in a portion of the video stream frames. One example of a software framework that enables the image processing operations described above in the application software 250 is the Open Computer Vision (OpenCV) project that is available at https://opencv.org/. The process described above for identification of the vial 160 is substantially the same as the processes described below for the identification of other objects in the video stream during the process 300.

During the vial identification process, the mobile electronic device 140 optionally transmits a graphic, such as an icon or animation, to the wearable electronic device 104 to assist the user in identifying the next step in the process for performing the test analysis. For example, the mobile electronic device 140 transmits a graphical icon that corresponds to the shape of the vial 160 to the wearable electronic device 104, and the wearable electronic device processor 204 generates a graphical display of the icon using the HUD 116 to alert the user to retrieve the vial 160 and place it in view of the camera 108 until successful identification of the vial 160 in the video stream. In FIG. 4, the view 404 depicts the icon 408 that the HUD 116 superimposes over the scene recorded by the camera 108 to prompt the user to retrieve the vial 160.

Referring again to FIG. 3, the process 300 continues as the mobile electronic device processor 224 identifies that the vial 160 is opened in response to detecting the registration mark formed 168 that is formed on the interior of the lid 164 in the vial 160 (block 312). Referring to FIG. 4, view 412 depicts the lid 164 removed from the vial 160 with the registration mark 168 visible on the interior of the lid 164. The mobile electronic device processor 224 tracks and identifies the lid 164 within the image segment 416 in the same manner that is described above regarding the vial 160. Additionally, the mobile electronic device 140 optionally transmits an icon of the lid to the wearable electronic device 104, and the wearable electronic device 104 displays the icon 420 in the HUD 116 to provide guidance to the user.

Referring again to FIG. 3 and FIG. 4, the process 300 continues as the mobile electronic device processor 224 identifies that the test strip 170 has been removed from the opened vial 160 (block 316). The mobile electronic device processor 224 tracks and identifies the test strip 170 within the image segment 428 in the same manner that is described above regarding the vial 160 and lid 164 as depicted in view 424. Additionally, the mobile electronic device 140 optionally transmits an icon of the test strip to the wearable electronic device 104, and the wearable electronic device 104 displays the icon 432 in the HUD 116 to provide guidance to the user. As depicted in FIG. 4, in some instances the user removes the test strip with the reverse side 170' visible to the camera 108. The mobile electronic device processor 224 tracks and identifies the test strip reverse side 170' in the region 430 and optionally generates an output message for the user via the HUD 116 or another output device 146 to rotate the test strip so that the side of the test strip 170 with the registration marks 174 and the deposit site 172 is visible in the video stream. The absence of the registration marks 174 on the test strip reverse side 170' and optionally other different features of the reverse side 170' provide sufficient differences for the image classifier to distinguish the sides 170/170' of the test strip. This operation is performed at subsequent stages of the process 300 if the test strip 170 is flipped to expose the reverse side 170' prior to the test strip 170 receiving the fluid dose. While view 424 depicts the vial 160, interior of the lid 164, and the test strip 170 concurrently for illustrative purposes, the detection of removal of the test strip 170 from the vial 160 only requires a sequence of detecting the vial 160, lid interior 164, and the test strip 170 within the same video stream within a comparatively short period of time, such as a 10 second, 30 second, or 60 second time window. As such, the vial 160, lid 164, and test strip 170 do not need to be identified simultaneously in the video stream for the process 300 to identify that the test strip 170 has been removed from the vial 160.

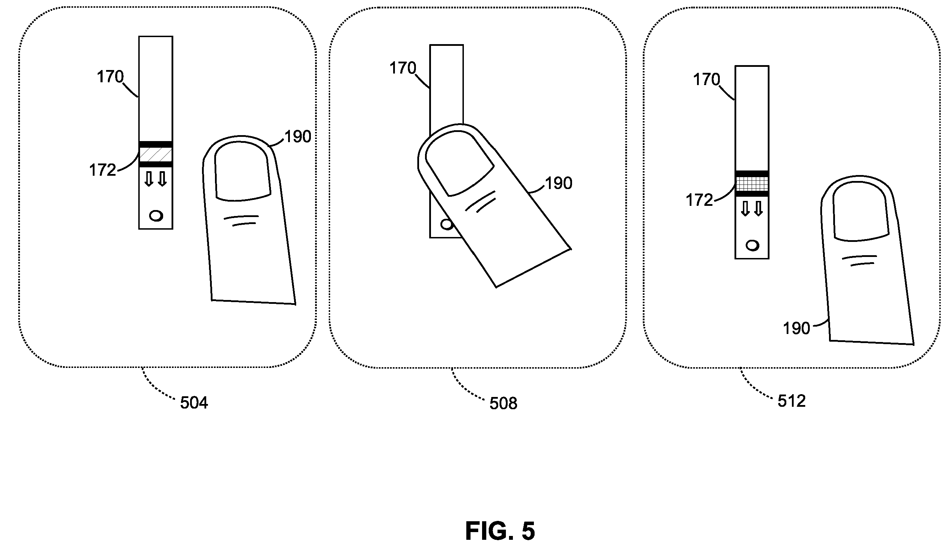

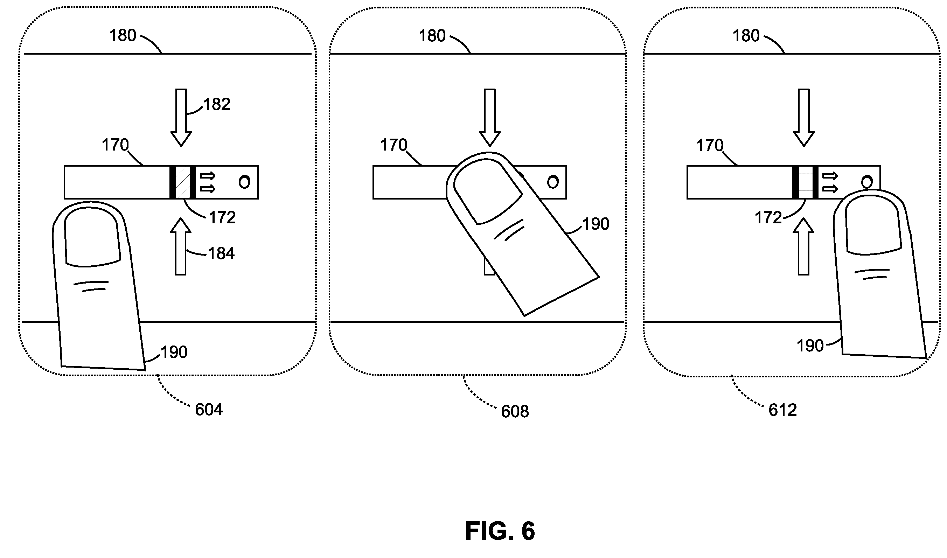

During the process 300, if the mobile electronic device processor 224 fails to identify the sequence of the vial 160, the interior of the lid 164 indicating that the vial 160 is opened, or the removal of the test strip 170 within a predetermined period of time (block 320) then the process 300 returns to the processing described above with reference to block 308 to enable the user to repeat the process. Upon successfully identification that the test strip 170 has been removed from the vial 160 (block 320) the process 300 continues as the mobile electronic device processor 224 continues to track the test strip 170 that has been identified in the video stream (block 328). In one configuration, the mobile electronic device processor 224 tracks the test strip 170 in isolation as depicted in view 504 of FIG. 5. In another configuration that utilizes the color card 180, the mobile electronic device processor 224 identifies the color card 180 based at least in part on the registration mark arrows 182 and 184 and tracks the insertion of the test strip 170 into the color card 180 as depicted in view 604 of FIG. 6. After identification of the test strip 170 and prior to the test strip 170 receiving the fluid dose, the mobile electronic device processor 224 stores at least one image of the test strip, including the deposit site 172, in the memory 232. As described in further detail below, at least one optical property of the deposit site 172 changes after the deposit site 172 receives the fluid dose, and the change in optical property enables detection of the time at which the test strip 170 receives the fluid dose.

The process 300 continues as the mobile electronic device processor 224 identifies the application of the fluid dose to the deposit site 172 on the test strip 170 based on the video stream, and starts the timer 226 upon identification of the fluid dose application (block 332). In one configuration, the mobile electronic device processor 224 identifies the finger 190 of the user in the video stream using the same procedure described above for identification of the vial 160, lid 164, test strip 170, and color card 180. The mobile electronic device processor 224 further identifies contact between the finger and the deposit site 172 in the video stream. For example, contact is identified in response to the finger 190 occluding the test strip 170 in the video stream as depicted in view 508 of FIG. 5 and view 608 of FIG. 6. After identification of the contact, the mobile electronic device processor 224 identifies that the fluid dose has been applied to the deposit side 172 based on a change in at least one optical property of the deposit site 172 in the video stream relative to the previously recorded images of the deposit site 172 as depicted in view 512 of FIG. 5 and view 612 of FIG. 6. Examples of optical properties of the deposit site 172 that change after the deposit site 172 receives the fluid dose include changes in one or more of color, contrast, and brightness of the deposit site 172 that occur due to the application of the fluid dose. In another configuration, the mobile electronic device processor 224 omits the identification of the finger 190 and contact between the finger 190 and the test strip 170 in the video stream. In this simplified configuration, the mobile electronic device processor 224 continues to track the test strip 170 until the detection of the change in the at least one optical property of the deposit site 172 to identify that the test strip 170 has received the fluid dose. In yet another configuration, the mobile electronic device processor 224 omits the identification of a change in the optical characteristic of the deposit site 172 and instead detects the dosing of the test strip 170 based on detection of contact between the finger 190 and the test strip 170 in the video stream. In this simplified configuration, the mobile electronic device processor 224 identifies contact based on the finger 190 occluding the test strip 170 in the video stream, such as in views 508 and 608, or on close proximity of the finger 190 and test strip 170. This configuration enables detection of test strip dosing in situations where poor ambient light conditions make detection of changes in the optical characteristic at the dosing site 172 difficult to detect. In all three configurations, the mobile electronic device processor 224 activates the timer 226 upon detection of the application of the fluid dose to the deposit site 172 on the test strip 170.

The process 300 continues as the timer 226 reaches a predetermined minimum time and the mobile electronic device processor 224 optionally generates an output signal to the user that the optical sensor 142 should be used to generate one or more optical measurements of the measurement site 178 on the test strip reverse side 170' (block 336). The mobile electronic device processor 224 generates an output on a display touchscreen 146 of the mobile electronic device 140 or via the HUD 116 of the wearable electronic device 104 to indicate that the optical measurement of the test strip should proceed as the optical sensor 142 generates one or more optical measurements of the measurement site 178 (block 340). The mobile electronic device processor 224 optionally generates an output that includes a countdown timer to indicate the amount of time remaining in the predetermined time window after the expiration of the minimum time period and prior to the expiration of the predetermined maximum time period to generate the optical measurements. In one configuration, the mobile electronic device processor 224 activates the optical sensor 142 only after the timer 226 indicates that the predetermined minimum time period has expired, while in another configuration the mobile electronic device processor 224 only accepts optical measurements from the optical sensor 142 that have a timestamp that falls within the predetermined time window. While the precise predetermined minimum and maximum time periods for generation of the optical measurements may vary between embodiments, in one configuration the minimum time period following the fluid dose is 13 seconds and the maximum time period is 45 seconds. This provides a 32 second time window for the optical sensor 142 to produce one or more optical measurements of the measurement site 178.

After the timer 226 reaches the expiration of the predetermined maximum time period, the mobile electronic device 224 generates an output to indicate that the maximum time period has expired (block 344). If a sufficient number of optical measurements have been generated prior to the expiration of the predetermined maximum time period (block 348), then the mobile electronic device processor 224 continues with the analyte measurement process based on the optical measurements (block 352). In another configuration, if the optical sensor 142 generates a sufficient number of optical measurements prior to the expiration of the predetermined maximum time period, then the mobile electronic device processor 224 optionally commences the measurement process of block 352 without waiting for the expiration of the timer 226. While not described in further detail herein, the analyte measurement process analyzes the color and optionally other optical properties of the reagent at the measurement site 178 on the test strip to determine the level of analyte in the fluid sample, such as the level of glucose in a blood sample. In configurations that use the color card 180, the mobile electronic device processor 224 uses additional optical data from the color card 180 to assist with the analyte measurement process. The mobile electronic device 140 displays the measurement of the analyte level to the user via the display device 146, the HUD 116 in the wearable electronic device 104, or via another output device. The system 100 and process 300 increase the reliability of the analyte measurement process because all of the optical measurements are generated during the predetermined time window to ensure that the reagents in the test strip 170 have sufficient time to complete chemical reactions prior to the generation of the optical measurements but also do not experience dehydration or bleaching before the completion of the optical measurement process.

During the process 300, if the predetermined maximum time period expires prior to the generation of a sufficient number of optical measurements (block 348), then the mobile electronic device processor 224 does not continue with the analyte measurement process and the mobile electronic device 140 generates an output message indicating that the analyte measurement cannot be completed and instructing the user begin the analyte testing process again using a new test strip via the display device 146, the HUD 116 in the wearable electronic device 104, or via another output device (block 356).

As described above, process 300 performs object identification that begins with identification of the test strip vial 160 and the opening of the lid 164 in the video stream, which enables the system 100 to verify that the test strip 170 was extracted from the vial 160 instead of being a loose test strip that may have been outside of the vial 160 for a prolonged period of time. Some test strips may be contaminated if left outside of a vial for a prolonged period of time. However, in a simplified configuration of the process 300, the system 100 omits the identification of the test strip vial 160, the lid 164, and the extraction of the test strip 170 from the vial 160. The simplified configuration begins with the generation of the video stream and the tracking and identification of the test strip 170 in the same manner that is described above. In this configuration, the process 300 does not verify that the test strip 170 was extracted from a vial, which may not be necessary for some analyte testing systems. This simplified configuration of the process 300 is otherwise identical to the process described above.

While the embodiments disclosed herein use a separate wearable electronic device 104 and mobile electronic device 140 for illustrative purposes, those of skill in the art will recognize that a single electronic device could be configured to perform the operations described herein. In particular, while state of the art wearable electronic devices typically interface with a mobile electronic device for complex operations, more capable wearable electronic devices could implement all of the functions described herein. Alternatively, the mobile electronic device 140 could be configured to perform all of the functions described herein using the optical sensor 142 as a camera to generate the video stream and perform the other processing that is described above. As such, specific references to the operations of a processor refer to the wearable electronic device processor 204 and the mobile electronic device processor 224 in the description above both individually, in combination, and, alternatively, to the operation of a single processor in configurations that use a single electronic device.

This disclosure is described in connection with what are considered to be the most practical and preferred embodiments. However, these embodiments are presented by way of illustration and are not intended to be limited to the disclosed embodiments. Accordingly, one of skill in the art will realize that this disclosure encompasses all modifications and alternative arrangements within the scope of the invention, as set forth in the following claims.

## Claims

1. A method for measuring an analyte comprising:
- identifying, with a processor (204), test strip (170) in a video stream generated by a camera (108) based on at least one registration mark (174) associated with the test strip depicted in the video stream;
- identifying, with the processor, application of a fluid dose to a deposit site (172) formed on the test strip based on the video stream;
- activating, with the processor, a timer (226) in response to the identification of the application of the fluid dose;
- generating, with an optical sensor (142), at least one optical measurement of a reagent located at a measurement site (178) on the test strip; and generating, with the processor, a measurement of an analyte in the fluid dose based on the at least one optical measurement of the reagent only in response to the at least one optical measurement being generated after a predetermined minimum time period has elapsed subsequent to the activating of the timer and prior to a predetermined maximum time period elapsing subsequent to the activating of the timer, **characterized in that** the method further comprises:
- identifying, with the processor, a vial (160) in a video stream generated by a camera based on at least one of an outline shape of the test vial or at least one registration mark (163) located on the vial depicted in the video stream;
- identifying, with the processor, an opening of the vial in the video stream based on at least one registration mark (168) located on a lid (164) of the vial; and
- identifying, with a processor, extraction of the test strip from the vial after the identifying of the opening of the vial in the video stream based on the at least one registration mark associated with the test strip depicted in the video stream.

2. The method of claim 1, wherein the at least one registration mark associated with the vial further comprises an indicator formed on a label (162) of the vial.

3. The method of claim 1 or 2, wherein the at least one registration mark located on the lid of the vial further comprises a color marking (168) formed on an interior surface of the lid.

4. The method of one of claims 1 to 3, wherein the at least one registration mark (163) associated with the test strip further comprises a printed mark formed on a side of the test strip at a predetermined position relative to the deposit site.

5. The method of claim 4 further comprising:
- identifying, with the processor, that a reverse side of the test strip is exposed in the video stream based on an absence of the printed mark formed on the side of the test strip; and
- generating, with the processor and an output device, an output message indicating that the test strip should be rotated to expose the side of the test strip bearing the printed mark.

6. The method of one of claims 1 to 5, wherein the at least one registration mark associated with the test strip further comprises an indicator formed on a rear surface of a color card that holds the test strip.

7. The method of one of claims 1 to 6, the identifying of the application of the dose further comprising:
- identifying, with the processor, a finger of a user in the video stream;
- identifying, with the processor, contact between the finger and the deposit site in the video stream; and
- identifying, with the processor, the application of the dose in response to a change in an optical property of the deposit site in the video stream after the contact between the finger and the deposit site.

8. The method of one of claims 1 to 7 the identifying of the application of the dose further comprising:
- identifying, with the processor, the application of the dose in response to a change in an optical property of the deposit site in the video stream.

9. The method of one of claims 1 to 8, the identifying of the application of the dose further comprising:
- identifying, with the processor, the application of the dose in response to contact between the finger and the deposit site in the video stream.

10. The method of one of claims 1 to 9 further comprising:
- generating, with the processor and an output device, an output message informing the user that the measurement of the analyte in the fluid dose cannot be completed in response to no optical measurement of the measurement site being generated after the predetermined minimum time period has elapsed and prior to the predetermined maximum time period elapsing.

11. The method of one of claims 1 to 10, wherein the optical sensor that generates the measurement is the camera that generates the video stream.

12. The method of one of claims 1 to 11, wherein the optical sensor that generates the measurement is a camera that is different than the camera that generates the video stream.

13. The method of claim 12, wherein the camera is incorporated in a wearable electronic device and the optical sensor is incorporated in a mobile electronic device.

14. A system for measurement of an analyte comprising:
a wearable electronic device (104) and a mobile electronic device (140), the wearable electronic
device comprising:
- a camera (142) configured to generate the video stream; and
- a transmitter configured to transmit the video stream to the mobile electronic device; and
the mobile electronic device comprising:
- a receiver configured to receive the video stream transmitted from the wearable electronic device;
- an optical sensor configured to generate optical measurements;
- a memory configured to store program instructions; and
- a processor operatively connected to the receiver, the optical sensor, and the memory, the processor being configured to execute the program instructions to:
- identify a test strip (170) in the video stream based on at least one registration mark (174) associated with the test strip depicted in the video stream;
- identify application of a fluid dose to a deposit site (172) formed on the test strip based on the video stream;
- activate a timer (226) in response to the application of the fluid dose;
- generate, with the optical sensor (142), at least one optical measurement of a reagent located at a measurement site (178) on the test strip; and
- generate a measurement of an analyte in the fluid dose based on the at least one optical measurement of the reagent only in response to the at least one optical measurement being generated after a predetermined minimum time period has elapsed subsequent to the activating of the timer and prior to a predetermined maximum time period elapsing subsequent to the activating of the timer,
**characterized in that**
the processor is further configured to:
- identify a vial (160) in the video stream generated by a camera based on at least one registration mark (163) located on the vial depicted in the video stream;
- identify an opening of the vial in the video stream based on at least one of an outline shape of the test vial or at least one registration mark (168) located on a lid (164) of the vial; and
- identify extraction of the test strip (170) from the vial (160) after the identification of the opening of the vial in the video stream based on the at least one registration mark (174) associated with the test strip depicted in the video stream.

15. The system of claim 14, wherein the at least one registration mark associated with the vial further comprises an indicator formed on a label (162) of the vial.

16. The system of claim 14 or 15, wherein the at least one registration mark (168) located on the lid of the vial further comprises a color marking formed on an interior surface of the lid.

17. The system of one of claims 14 to 16, wherein the at least one registration mark associated with the test strip further comprises an indicator formed on a surface of the test strip at a predetermined position relative to the deposit site.

18. The system claim 17, the processor being further configured to:
- identify that a reverse side of the test strip is exposed in the video stream based on an absence of the indicator formed on the surface of the test strip; and
- generate, with an output device, an output message indicating that the test strip should be rotated to expose the surface of the test strip bearing the indicator.

19. The system of one of claims 14 to 18, wherein the at least one registration mark associated with the test strip further comprises an indicator formed on a rear surface of a color card that holds the test strip.

20. The system of one of claims 14 to 19, the processor being further configured to:
- identify a finger of a user in the video stream;
- identify contact between the finger and the deposit site in the video stream; and
- identify the application of the dose in response to a change in an optical property of the deposit site in the video stream after the contact between the finger and the deposit site

21. The system of one of claims 14 to 20 the processor being further configure to:
- identify the application of the dose in response to a change in an optical property of the deposit site in the video stream.

22. The system of one of claims 14 to 21 the processor being further configure to:
- identify the application of the dose in response to contact between the finger and the deposit site in the video stream.

23. The system of one of claims 14 to 22 further comprising:
- an output device in at least one of the wearable electronic device or the mobile electronic device, the processor being operatively connected to the output device and further configured to:
- generate an output message informing the user that the measurement of the analyte in the fluid dose cannot be completed in response to no optical measurement of the measurement site being generated after the predetermined minimum time period has elapsed and prior to the predetermined maximum time period elapsing.

## Patentansprüche

1. Verfahren zum Messen eines Analyten, umfassend:
- Erkennen eines Teststreifens (170) in einem Videostream, der von einer Kamera (108) erzeugt wird, mit einem Prozessor (204) basierend auf mindestens einer Registriermarkierung (174) in Verbindung mit dem in dem Videostream gezeigten Teststreifen;
- Erkennen der Aufbringung einer Fluiddosis auf eine Abscheidungsstelle (172), die auf dem Teststreifen gebildet ist, mit dem Prozessor basierend auf dem Videostream;
- Aktivieren eines Timers (226) mit dem Prozessor als Reaktion auf die Erkennung der Aufbringung der Fluiddosis;
- Erzeugen mindestens einer optischen Messung eines Reagens, das sich an einer Messstelle (178) auf dem Teststreifen befindet, mit einem optischen Sensor (142); und Erzeugen einer Messung eines Analyten in der Fluiddosis mit dem Prozessor basierend auf der mindestens einen optischen Messung des Reagens nur als Reaktion darauf, dass die mindestens eine optische Messung erzeugt wird, nachdem eine vorbestimmte Mindestzeitdauer nach der Aktivierung des Timers abgelaufen ist und bevor eine vorbestimmte Höchstzeitdauer nach der Aktivierung des Timers abläuft, **dadurch gekennzeichnet, dass** das Verfahren ferner Folgendes umfasst:
- Erkennen eines Fläschchens (160) in einem von einer Kamera erzeugten Videostream mit dem Prozessor basierend auf mindestens einem von einer Umrissform des Testfläschchens oder mindestens einer Registriermarkierung (163), die sich auf dem in dem Videostream gezeigten Fläschchen befindet;
- Erkennen einer Öffnung des Fläschchens in dem Videostream mit dem Prozessor basierend auf mindestens einer Registriermarkierung (168), die sich auf einem Deckel (164) des Fläschchens befindet; und
- Erkennen der Entnahme des Teststreifens aus dem Fläschchen mit dem Prozessor nach dem Erkennen der Öffnung des Fläschchens in dem Videostream basierend auf der mindestens einen Registriermarkierung in Verbindung mit dem Teststreifen, der in dem Videostream gezeigt wird.

2. Verfahren nach Anspruch 1, wobei die mindestens eine Registriermarkierung in Verbindung mit dem Fläschchen ferner einen auf einem Etikett (162) des Fläschchens gebildeten Indikator umfasst.

3. Verfahren nach Anspruch 1 oder 2, wobei die mindestens eine Registriermarkierung, die sich auf dem Deckel des Fläschchens befindet, ferner eine Farbmarkierung (168) umfasst, die auf einer Innenfläche des Deckels gebildet ist.

4. Verfahren nach einem der Ansprüche 1 bis 3, wobei die mindestens eine Registriermarkierung (163) in Verbindung mit dem Teststreifen ferner eine gedruckte Markierung umfasst, die auf einer Seite des Teststreifens an einer vorbestimmten Position in Bezug auf die Abscheidungsstelle gebildet ist.

5. Verfahren nach Anspruch 4, ferner umfassend:
- Erkennen, dass eine Rückseite des Teststreifens in dem Videostream freiliegt, mit dem Prozessor basierend auf der Abwesenheit der gedruckten Markierung, die auf der Seite des Teststreifens gebildet ist; und
- Erzeugen einer Ausgabenachricht, die angibt, dass der Teststreifen gedreht werden sollte, um die Seite des Teststreifens freizulegen, die die gedruckte Markierung trägt, mit dem Prozessor und einer Ausgabevorrichtung.

6. Verfahren nach einem der Ansprüche 1 bis 5, wobei die mindestens eine Registriermarkierung in Verbindung mit dem Teststreifen ferner einen Indikator umfasst, der auf einer hinteren Fläche einer Farbkarte gebildet ist, die den Teststreifen hält.

7. Verfahren nach einem der Ansprüche 1 bis 6, wobei das Erkennen der Aufbringung der Dosis ferner Folgendes umfasst:
- Erkennen eines Fingers eines Benutzers in dem Videostream mit dem Prozessor;
- Erkennen eines Kontakts zwischen dem Finger und der Abscheidungsstelle in dem Videostream mit dem Prozessor; und
- Erkennen der Aufbringung der Dosis als Reaktion auf eine Veränderung einer optischen Eigenschaft der Abscheidungsstelle in dem Videostream nach dem Kontakt zwischen dem Finger und der Abscheidungsstelle mit dem Prozessor.

8. Verfahren nach einem der Ansprüche 1 bis 7, wobei das Erkennen der Aufbringung der Dosis ferner Folgendes umfasst:
- Erkennen der Aufbringung der Dosis als Reaktion auf eine Veränderung einer optischen Eigenschaft der Abscheidungsstelle in dem Videostream mit dem Prozessor.

9. Verfahren nach einem der Ansprüche 1 bis 8, wobei das Erkennen der Aufbringung der Dosis ferner Folgendes umfasst:
- Erkennen der Aufbringung der Dosis als Reaktion auf einen Kontakt zwischen dem Finger und der Abscheidungsstelle in dem Videostream mit dem Prozessor.

10. Verfahren nach einem der Ansprüche 1 bis 9, ferner umfassend:
- Erzeugen einer Ausgabenachricht, die den Benutzer darüber informiert, dass die Messung des Analyten in der Fluiddosis nicht abgeschlossen werden kann, als Reaktion darauf, dass keine optische Messung der Messstelle erzeugt wird, nachdem die vorbestimmte Mindestzeitdauer abgelaufen ist und bevor die vorbestimmte Höchstzeitdauer abläuft, mit dem Prozessor und einer Ausgabevorrichtung.

11. Verfahren nach einem der Ansprüche 1 bis 10, wobei der optische Sensor, der die Messung erzeugt, die Kamera ist, die den Videostream erzeugt.

12. Verfahren nach einem der Ansprüche 1 bis 11, wobei der optische Sensor, der die Messung erzeugt, eine Kamera ist, die eine andere ist als die Kamera, die den Videostream erzeugt.

13. Verfahren nach Anspruch 12, wobei die Kamera in eine tragbare elektronische Vorrichtung eingebaut ist und der optische Sensor in eine mobile elektronische Vorrichtung eingebaut ist.

14. System zur Messung eines Analyten, umfassend:
eine tragbare elektronische Vorrichtung (104) und eine mobile elektronische Vorrichtung (140), wobei die tragbare elektronische Vorrichtung Folgendes umfasst:
- eine Kamera (142), die dafür ausgebildet ist, den Videostream zu erzeugen; und
- einen Sender, der dafür ausgebildet ist, den Videostream an die mobile elektronische Vorrichtung zu senden; und
wobei die mobile elektronische Vorrichtung Folgendes umfasst:
- einen Empfänger, der dafür ausgebildet ist, den aus der tragbaren elektronischen Vorrichtung gesendeten Videostream zu empfangen;
- einen optischen Sensor, der dafür ausgebildet ist, optische Messungen zu erzeugen;
- einen Speicher, der dafür ausgebildet ist, Programmanweisungen zu speichern; und
- einen Prozessor, der funktionsfähig an den Empfänger, den optischen Sensor und den Speicher angeschlossen ist, wobei der Prozessor dafür ausgebildet ist, die Programmanweisungen auszuführen, um:
- einen Teststreifen (170) in dem Videostream basierend auf mindestens einer Registriermarkierung (174) in Verbindung mit dem Teststreifen, der in dem Videostream gezeigt wird, zu erkennen;
- die Aufbringung einer Fluiddosis auf eine Abscheidungsstelle (172), die auf dem Teststreifen gebildet ist, basierend auf dem Videostream zu erkennen;
- einen Timer (226) als Reaktion auf die Aufbringung der Fluiddosis zu aktivieren;
- mit dem optischen Sensor (142) mindestens eine optische Messung eines Reagens, das sich an einer Messstelle (178) auf dem Teststreifen befindet, zu erzeugen; und
- eine Messung eines Analyten in der Fluiddosis basierend auf der mindestens einen optischen Messung des Reagens nur als Reaktion darauf, dass die mindestens eine optische Messung erzeugt wird, nachdem eine vorbestimmte Mindestzeitdauer nach dem Aktivieren des Timers abgelaufen ist und bevor eine vorbestimmte Höchstzeitdauer nach dem Aktivieren des Timers abläuft, zu erzeugen, **dadurch gekennzeichnet, dass** der Prozessor ferner zu Folgendem ausgebildet ist:
- Erkennen eines Fläschchens (160) in dem von einer Kamera erzeugten Videostream basierend auf mindestens einer Registriermarkierung (163), die sich auf dem in dem Videostream gezeigten Fläschchen befindet;
- Erkennen einer Öffnung des Fläschchens in dem Videostream basierend auf mindestens einem von einer Umrissform des Testfläschchens oder mindestens einer Registriermarkierung (168), die sich auf dem Deckel (164) des Fläschchens befindet; und
- Erkennen der Entnahme des Teststreifens (170) aus dem Fläschchen (160) nach der Erkennung der Öffnung des Fläschchens in dem Videostream basierend auf der mindestens einen Registriermarkierung (174) in Verbindung mit dem Teststreifen, der in dem Videostream gezeigt wird.

15. System nach Anspruch 14, wobei die mindestens eine Registriermarkierung in Verbindung mit dem Fläschchen ferner einen auf einem Etikett (162) des Fläschchens gebildeten Indikator umfasst.

16. System nach Anspruch 14 oder 15, wobei die mindestens eine Registriermarkierung (168), die sich auf dem Deckel des Fläschchens befindet, ferner eine Farbmarkierung umfasst, die auf einer Innenfläche des Deckels gebildet ist.

17. System nach einem der Ansprüche 14 bis 16, wobei die mindestens eine Registriermarkierung in Verbindung mit dem Teststreifen ferner einen Indikator umfasst, der auf einer Fläche des Teststreifens an einer vorbestimmten Position in Bezug auf die Abscheidungsstelle gebildet ist.

18. System nach Anspruch 17, wobei der Prozessor ferner zu Folgendem ausgebildet ist:
- Erkennen, dass eine Rückseite des Teststreifens in dem Videostream freiliegt, basierend darauf, dass kein Indikator auf der Fläche des Teststreifens gebildet ist; und
- Erzeugen einer Ausgabenachricht, die angibt, dass der Teststreifen gedreht werden sollte, um die Fläche des Teststreifens freizulegen, die den Indikator trägt, mit einer Ausgabevorrichtung.

19. System nach einem der Ansprüche 14 bis 18, wobei die mindestens eine Registriermarkierung in Verbindung mit dem Teststreifen ferner einen Indikator umfasst, der auf einer hinteren Fläche einer Farbkarte gebildet ist, die den Teststreifen hält.

20. System nach einem der Ansprüche 14 bis 19, wobei der Prozessor ferner zu Folgendem ausgebildet ist:
- Erkennen eines Fingers eines Benutzers in dem Videostream;
- Erkennen eines Kontakts zwischen dem Finger und der Abscheidungsstelle in dem Videostream; und
- Erkennen der Aufbringung der Dosis als Reaktion auf eine Veränderung einer optischen Eigenschaft der Abscheidungsstelle in dem Videostream nach dem Kontakt zwischen dem Finger und der Abscheidungsstelle.

21. System nach einem der Ansprüche 14 bis 20, wobei der Prozessor ferner zu Folgendem ausgebildet ist:
- Erkennen der Aufbringung der Dosis als Reaktion auf eine Veränderung einer optischen Eigenschaft der Abscheidungsstelle in dem Videostream.

22. System nach einem der Ansprüche 14 bis 21, wobei der Prozessor ferner zu Folgendem ausgebildet ist:
- Erkennen der Aufbringung der Dosis als Reaktion auf einen Kontakt zwischen dem Finger und der Abscheidungsstelle in dem Videostream.

23. System nach einem der Ansprüche 14 bis 22, ferner umfassend:
- eine Ausgabevorrichtung in mindestens einer von der tragbaren elektronischen Vorrichtung oder der mobilen elektronischen Vorrichtung, wobei der Prozessor funktionsfähig an die Ausgabevorrichtung angeschlossen und ferner zu Folgendem ausgebildet ist:
- Erzeugen einer Ausgabenachricht, die den Benutzer darüber informiert, dass die Messung des Analyten in der Fluiddosis nicht abgeschlossen werden kann, als Reaktion darauf, dass keine optische Messung der Messstelle erzeugt wird, nachdem die vorbestimmte Mindestzeitdauer abgelaufen ist und bevor die vorbestimmte Höchstzeitdauer abläuft.

## Revendications

1. Procédé de mesure d'un analyte comprenant :
- l'identification, à l'aide d'un processeur (204), d'une bandelette de test (170) dans un flux vidéo généré par une caméra (108) sur la base d'au moins une marque d'enregistrement (174) associée à la bandelette de test illustrée dans le flux vidéo ;
- l'identification, à l'aide du processeur, de l'application d'une dose de fluide sur un site de dépôt (172) formé sur la bandelette de test sur la base du flux vidéo ;
- l'activation, à l'aide du processeur, d'un chronomètre (226) en réponse à l'identification de l'application de la dose de fluide ;
- la génération, à l'aide d'un capteur optique (142), d'au moins une mesure optique d'un réactif situé au niveau d'un site de mesure (178) sur la bandelette de test ; et la génération, à l'aide du processeur, d'une mesure d'un analyte dans la dose de fluide sur la base de l'au moins une mesure optique du réactif uniquement en réponse à la génération de l'au moins une mesure optique après qu'une période de temps minimale prédéterminée se soit écoulée suite à l'activation du chronomètre et avant qu'une période de temps maximale prédéterminée se soit écoulée suite à l'activation du chronomètre, **caractérisé en ce que** le procédé comprend en outre :
- l'identification, à l'aide du processeur, d'un flacon (160) dans un flux vidéo généré par une caméra sur la base d'au moins l'une parmi une forme de contour du flacon de test ou au moins une marque d'enregistrement (163) située sur le flacon illustré dans le flux vidéo ;
- l'identification, à l'aide du processeur, d'une ouverture du flacon dans le flux vidéo sur la base d'au moins une marque d'enregistrement (168) située sur un couvercle (164) du flacon ; et
- l'identification, à l'aide d'un processeur, de l'extraction de la bandelette de test du flacon après l'identification de l'ouverture du flacon dans le flux vidéo sur la base de l'au moins une marque d'enregistrement associée à la bandelette de test illustrée dans le flux vidéo.

2. Procédé selon la revendication 1, dans lequel l'au moins une marque d'enregistrement associée au flacon comprend en outre un indicateur formé sur une étiquette (162) du flacon.

3. Procédé selon la revendication 1 ou 2, dans lequel l'au moins une marque d'enregistrement située sur le couvercle du flacon comprend en outre un marquage couleur (168) formé sur une surface intérieure du couvercle.

4. Procédé selon l'une des revendications 1 à 3, dans lequel l'au moins une marque d'enregistrement (163) associée à la bandelette de test comprend en outre une marque imprimée formée sur un côté de la bandelette de test au niveau d'une position prédéterminée par rapport au site de dépôt.

5. Procédé selon la revendication 4 comprenant en outre :
- l'identification, à l'aide du processeur, qu'un verso de la bandelette de test est exposé dans le flux vidéo sur la base d'une absence de la marque imprimée formée sur le côté de la bandelette de test ; et
- la génération, à l'aide du processeur et d'un dispositif de sortie, d'un message de sortie indiquant que la bandelette de test devrait être retournée pour exposer le côté de la bandelette de test portant la marque imprimée.

6. Procédé selon l'une des revendications 1 à 5, dans lequel l'au moins une marque d'enregistrement associée à la bandelette de test comprend en outre un indicateur formé sur une surface arrière d'une carte de couleur qui retient la bandelette de test.

7. Procédé selon l'une des revendications 1 à 6, l'identification de l'application de la dose comprenant en outre :
- l'identification, à l'aide du processeur, d'un doigt d'un utilisateur dans le flux vidéo ;
- l'identification, à l'aide du processeur, d'un contact entre le doigt et le site de dépôt dans le flux vidéo ; et
- l'identification, à l'aide du processeur, de l'application de la dose en réponse à une modification d'une propriété optique du site de dépôt dans le flux vidéo après le contact entre le doigt et le site de dépôt.

8. Procédé selon l'une des revendications 1 à 7, l'identification de l'application de la dose comprenant en outre :
- l'identification, à l'aide du processeur, de l'application de la dose en réponse à une modification d'une propriété optique du site de dépôt dans le flux vidéo.

9. Procédé selon l'une des revendications 1 à 8, l'identification de l'application de la dose comprenant en outre :
- l'identification, à l'aide du processeur, de l'application de la dose en réponse à un contact entre le doigt et le site de dépôt dans le flux vidéo.

10. Procédé selon l'une des revendications 1 à 9 comprenant en outre :
- la génération, à l'aide du processeur et d'un dispositif de sortie, d'un message de sortie informant l'utilisateur que la mesure de l'analyte dans la dose de fluide ne peut pas être achevée en réponse à l'absence de génération de mesure optique du site de mesure après que la période de temps minimale prédéterminée se soit écoulée et avant que la période de temps maximale prédéterminée se soit écoulée.

11. Procédé selon l'une des revendications 1 à 10, dans lequel le capteur optique qui génère la mesure est la caméra qui génère le flux vidéo.

12. Procédé selon l'une des revendications 1 à 11, dans lequel le capteur optique qui génère la mesure est une caméra qui est différente de la caméra qui génère le flux vidéo.

13. Procédé selon la revendication 12, dans lequel la caméra est incorporée dans un dispositif électronique pouvant être porté et le capteur optique est incorporé dans un dispositif électronique mobile.

14. Système de mesure d'un analyte comprenant :
un dispositif électronique pouvant être porté (104) et un dispositif électronique mobile (140), le dispositif électronique pouvant être porté comprenant :
- une caméra (142) configurée pour générer le flux vidéo ; et
- un transmetteur configuré pour transmettre le flux vidéo au dispositif électronique mobile ; et
le dispositif électronique mobile comprenant :
- un récepteur configuré pour recevoir le flux vidéo transmis à partir du dispositif électronique pouvant être porté ;
- un capteur optique configuré pour générer des mesures optiques ;
- une mémoire configurée pour stocker des instructions de programme ; et
- un processeur connecté de manière fonctionnelle au récepteur, au capteur optique et à la mémoire, le processeur étant configuré pour exécuter les instructions de programme pour :
- identifier une bandelette de test (170) dans le flux vidéo sur la base d'au moins une marque d'enregistrement (174) associée à la bandelette de test illustrée dans le flux vidéo ;
- identifier l'application d'une dose de fluide sur un site de dépôt (172) formé sur la bandelette de test sur la base du flux vidéo ;
- activer un chronomètre (226) en réponse à l'application de la dose de fluide ;
- générer, à l'aide du capteur optique (142), au moins une mesure optique d'un réactif situé au niveau d'un site de mesure (178) sur la bandelette de test ; et
- générer une mesure d'un analyte dans la dose de fluide sur la base de l'au moins une mesure optique du réactif uniquement en réponse à la génération de l'au moins une mesure optique après qu'une période de temps minimale prédéterminée se soit écoulée suite à l'activation du chronomètre et avant qu'une période de temps maximale prédéterminée se soit écoulée suite à l'activation du chronomètre,
**caractérisé en ce que** le processeur est en outre configuré pour :
- identifier un flacon (160) dans le flux vidéo généré par une caméra sur la base d'au moins une marque d'enregistrement (163) située sur le flacon illustré dans le flux vidéo ;
- identifier une ouverture du flacon dans le flux vidéo sur la base d'au moins l'une parmi une forme de contour du flacon de test ou au moins une marque d'enregistrement (168) située sur un couvercle (164) du flacon ; et
- identifier l'extraction de la bandelette de test (170) du flacon (160) après l'identification de l'ouverture du flacon dans le flux vidéo sur la base de l'au moins une marque d'enregistrement (174) associée à la bandelette de test illustrée dans le flux vidéo.

15. Système selon la revendication 14, dans lequel l'au moins une marque d'enregistrement associée au flacon comprend en outre un indicateur formé sur une étiquette (162) du flacon.

16. Système selon la revendication 14 ou 15, dans lequel l'au moins une marque d'enregistrement (168) située sur le couvercle du flacon comprend en outre un marquage couleur formé sur une surface intérieure du couvercle.

17. Système selon l'une des revendications 14 à 16, dans lequel l'au moins une marque d'enregistrement associée à la bandelette de test comprend en outre un indicateur formé sur une surface de la bandelette de test au niveau d'une position prédéterminée par rapport au site de dépôt.

18. Système selon la revendication 17, le processeur étant en outre configuré pour :
- identifier qu'un verso de la bandelette de test est exposé dans le flux vidéo sur la base d'une absence de l'indicateur formé sur la surface de la bandelette de test ; et
- générer, à l'aide d'un dispositif de sortie, un message de sortie indiquant que la bandelette de test devrait être retournée pour exposer la surface de la bandelette de test portant l'indicateur.

19. Système selon l'une des revendications 14 à 18, dans lequel l'au moins une marque d'enregistrement associée à la bandelette de test comprend en outre un indicateur formé sur une surface arrière d'une carte de couleur qui retient la bandelette de test.

20. Système selon l'une des revendications 14 à 19, le processeur étant en outre configuré pour :
- identifier un doigt d'un utilisateur dans le flux vidéo ;
- identifier un contact entre le doigt et le site de dépôt dans le flux vidéo ; et
- identifier l'application de la dose en réponse à une modification d'une propriété optique du site de dépôt dans le flux vidéo après le contact entre le doigt et le site de dépôt.

21. Système selon l'une des revendications 14 à 20, le processeur étant en outre configuré pour :
- identifier l'application de la dose en réponse à une modification d'une propriété optique du site de dépôt dans le flux vidéo.

22. Système selon l'une des revendications 14 à 21, le processeur étant en outre configuré pour :
- identifier l'application de la dose en réponse à un contact entre le doigt et le site de dépôt dans le flux vidéo.

23. Système selon l'une des revendications 14 à 22 comprenant en outre :
- un dispositif de sortie dans au moins l'un parmi le dispositif électronique pouvant être porté ou le dispositif électronique mobile, le processeur étant connecté de manière fonctionnelle au dispositif de sortie et étant en outre configuré pour :
- générer un message de sortie informant l'utilisateur que la mesure de l'analyte dans la dose de fluide ne peut pas être achevée en réponse à l'absence de génération de mesure optique du site de mesure après que la période de temps minimale prédéterminée se soit écoulée et avant que la période de temps maximale prédéterminée se soit écoulée.
